# NEW EUROPEAN PATENT SPECIFICATION

(11) **EP 2 845 573 B2**
(45) Date of publication and mention of the opposition decision: **11.12.2019**
(45) Mention of the grant of the patent: 12.10.2016
(21) Application number: 14190199.1
(22) Date of filing: 14.04.2005
(51) Int. Cl.: A61F 13/15

(54) **Dual cuff for a unitary disposable absorbent article made of a continuous cuff material**
Zweifache Stulpe für einen einheitlichen saugfähigen Einwegartikel aus durchgehendem Manschettenmaterial
Double revers pour article absorbant unitaire jetable fabriqué à partir d'un matériau continu de revers

(30) Priority: 14.04.2004 US 824122
(43) Date of publication of application: 11.03.2015
(62) Divisional of application: 09009727.0
(73) Proprietor: The Procter & Gamble Company, Cincinnati, OH 45202 (US)
(72) Inventor: Ashton, Gregory, Venice, 34293 (US); Fukuda, Eiro, Cincinnati, OH Ohio 45202 (US); Nishikawa, Masaharu, Cincinnati, OH Ohio 45202 (US)
(74) Representative: Elkington and Fife LLP

(56) References cited:
- WO-A-00/07534
- WO-A-94/18927
- WO-A-96/05792
- WO-A-97/20532
- WO-A-99/20215
- WO-A1-03/053320
- US-A- 4 795 454

## Description

### FIELD OF THE INVENTION

This invention relates to dual cuffs on absorbent articles, and more particularly to dual cuffs having a first cuff and a second cuff on disposable absorbent articles.

### BACKGROUND OF THE INVENTION

The major function of absorbent articles, such as disposable diapers and adult incontinent briefs, is to absorb and contain body exudates. Such articles are thus intended to prevent body exudates from soiling, wetting, or otherwise contaminating clothing or other articles, such as bedding, that come in contact with the wearer. One common mode of failure for such products occurs when body exudates leak out of the gaps between the article and the wearer's leg to adjacent clothing because they are not immediately absorbed within the article. For example, urine tends to wick through the topsheet to the edges of the absorbent article where it can come in contact with clothing or other articles. Additionally, loose fecal material that is not easily absorbed by the absorbent article tends to "float" on the liquid-receiving surface and work its way past the legs of the wearer.

Contemporary absorbent articles have a topsheet, a backsheet, an absorbent core, a barrier cuff and a gasketing cuff. The gasketing cuff proves effective generally to prevent wicking and overflow from the fluid laden article to clothing contacting the edges of the article in that the gasketing cuff presents a fluid impermeable barrier between the edge of the article and the contacting clothing, and in addition, provides a gasketing action about the legs of the wearer. The barrier cuff proves effective generally to inhibit loose fecal material or gushes of urine or liquids from soiling the wearer's clothing. The barrier cuff restrains the free flow of this material and provides a structure to hold such material within the article.

While providing an inner and an outer cuff within an absorbent article has its advantages, the resulting article is often too wide in the crotch area, thus causing discomfort for the wearer. Furthermore, the outer cuff typically has an unfinished appearance that further adds to the perception of discomfort. Therefore, what is needed is an absorbent article that provides both effectiveness and comfort in the cuff region.

WO 00/07534 and WO 99/20215 disclose absorbent articles having, on each side of the article, a separately-provided gusset flap composite member. The gusset flap composite member provides a leg gusset section and a containment section to improve leakage resistance and comfort. The leg gusset extends the side edge contours of the backsheet layer.

WO 96/05792 discloses an absorbent article having, on each side, a stand-up barrier cuff and a stretchable leg cuff which extends in the plane of the article.

WO 03/053320 A1 discloses an absorbent article having, on each side, inner and outer containment flaps. The inner and outer flaps are constructed from a single web of material.

### SUMMARY OF THE INVENTION

The invention provides an absorbent article according to claim 1. Optional features of the absorbent article of the invention are set out in the dependent claims.

The absorbent article may be a prefastened diaper.

### BRIEF DESCRIPTION OF THE DRAWINGS

While the specification concludes with claims pointing out and distinctly claiming the present invention, it is believed the same will be better understood by the following drawings taken in conjunction with the accompanying specification wherein like components are given the same reference number.
FIG. 1 is a plan view of a disposable diaper embodiment of the present invention having portions cut-away to reveal underlying structure;
FIG. 2 is a fragmentary sectional view taken along section line 2-2 of FIG. 1;
FIG. 3a is a top view taken within encircled portion 3 of FIG. 1 illustrating a prior art example of a dual cuff within the crotch region;
FIG. 3b is a cross-sectional view of FIG. 3a;
FIG. 4a is a top view taken within encircled portion 3 of FIG. 1 illustrating an absorbent article having a dual cuff in accordance with the present invention within the crotch region;
FIG. 4b is a cross-sectional view of FIG. 4a;
FIG. 5 is a fragmentary sectional view taken along section line 5-5 of FIG. 1;
FIG. 6 is a flagmentary sectional view of another embodiment taken along section line 2-2 of FIG. 1;
FIG. 7 is a fragmentary sectional view of yet another embodiment taken along section line 2-2 of FIG. 1;
FIG. 8 is a fragmentary sectional view of yet another embodiment taken along section line 2-2 of FIG. 1; and
FIG. 9 is a fragmentary sectional view of yet another embodiment taken along section line 2-2 of FIG. 1;
FIG. 10a is a close-up view taken within encircled portion 10 of FIG. 2 illustrating a fold-over technique of cuff material, wherein a lower cuff material end is positioned longitudinally outboard of cuff bond; and
FIG. 10b is a close-up view taken within encircled portion 10 of FIG. 2 illustrating a fold-over technique of cuff material, wherein a lower cuff material end is positioned longitudinally inboard of cuff bond.

### DETAILED DESCRIPTION OF THE INVENTION

As used herein, the following terms have the following meanings:
"Absorbent article" refers to devices that absorb and contain liquid, and more specifically, refers to devices that are placed against or in proximity to the body of the wearer to absorb and contain the various exudates discharged from the body.
"Longitudinal" is a direction running parallel to the maximum linear dimension of the article and includes directions within ±45° of the longitudinal direction.

The "lateral" or "transverse" direction is orthogonal to the longitudinal direction.

The "Z-direction" is orthogonal to both the longitudinal and transverse directions.

The "x-y plane refers to the plane congruent with the longitudinal and transverse directions.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "disposed" is used to mean that an element(s) is formed (joined and positioned) in a particular place or position as a unitary structure with other elements or as a separate element joined to another element.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

A "unitary" absorbent article refers to absorbent articles which are formed of separate parts united together to form a coordinated entity so that they do not require separate manipulative parts like a separate holder and liner.

As used herein, the term "diaper" refers to an absorbent article generally worn by infants and incontinent persons about the lower torso.

The terms "water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water to pass through its thickness in the absence of a forcing pressure. Conversely, the term "water-impermeable" refers to a layer or a layered structure through the thickness of which liquid water cannot pass in the absence of a forcing pressure. A layer or a layered structure that is water-impermeable according to this definition may be permeable to water vapor, *i.e.*, may be "vapor-permeable". As is well known in the art, a common method for measuring the permeability to water of the materials typically used in absorbent articles is a hydrostatic pressure test, also called a hydrostatic head test or simply a "hydrohead" test. Suitable well known compendial method for hydrohead testing are approved by INDA (formerly the International Nonwovens and Disposables Association, now The Association of the Nonwoven Fabrics Industry) and EDANA (European Disposables And Nonwovens Association).

The terms "proximal" and "distal" refer respectively to the location of an element near to or far from the center of a structure, e.g., the proximal edge of a longitudinally extending element is located nearer to the longitudinal axis than the distal edge of the same element is located relative to the same longitudinal axis.

As used herein, the term "barrier cuff" refers to an elasticized flap which stands substantially upright, more preferably inwardly towards the longitudinal centerline, within the crotch region. Typically, said barrier cuff envelopes/contains at least one elastic that is connected primarily at its opposing ends to the diaper (e.g., drawstring technique for better fit).

As used herein, the term "gasketing cuff" refers to an elasticized flap which does not stand substantially upright, more preferably outwardly towards the longitudinal side edges of the diaper, within the crotch region. Typically, said gasketing cuff envelopes/contains at least one elastic that is connected substantially throughout its length to the diaper (e.g., multiple bonds along length of elastic to create gathers).

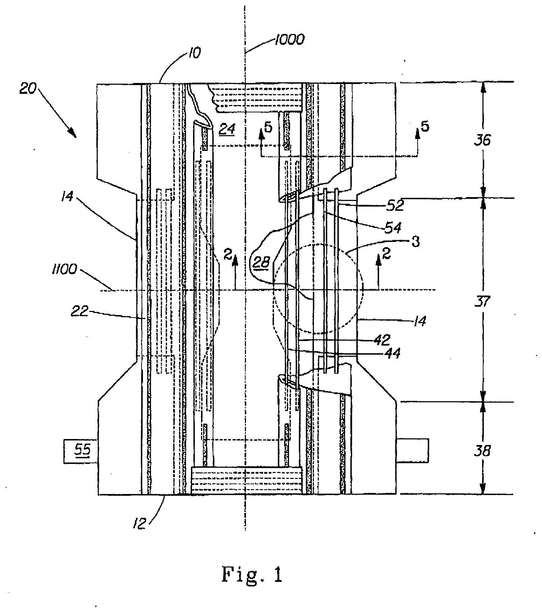

Figure 1 is a plan view of an exemplary, non-limiting embodiment of a diaper 20 of the present invention in its flat-out, uncontracted state (i.e., without elastic induced contraction) with portions of the structure being cut away to more clearly show the underlying structure of the diaper 20 and with the portion of the diaper 20 which contacts the wearer facing the viewer. The diaper 20 includes a longitudinal axis 1000 and a lateral axis 1100. One end portion 36 of the diaper 20 is configured as a first waist region 36 of the diaper 20. The opposite end portion 38 is configured as a second waist region 38 of the diaper 20. An intermediate portion 37 of the diaper 20 is configured as a crotch region 37, which extends longitudinally between the first and second waist regions 36 and 38. The waist regions 36 and 38 generally comprise those portions of the diaper 20 which, when worn, encircle the waist of the wearer. The waist regions 36 and 38 may include elastic elements such that they gather about the waist of the wearer to provide improved fit and containment The crotch region 37 is that portion of the diaper 20 which, when the diaper 20 is worn, is generally positioned between the legs of the wearer. The outer periphery of diaper 20 is defined by longitudinal edges 14 and end edges 10, 12 which are located along the first and second waist region 36, 38, respectively.

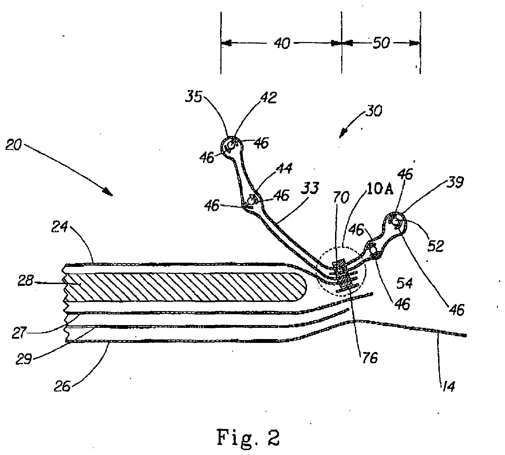

The chassis 22 of the diaper 20 comprises the main body of the diaper 20. The chassis 22 comprises an outer covering including a liquid permeable topsheet 24 and/or a liquid impermeable backsheet 26 (see FIG. 2) and at least a portion of an absorbent core 28 encased between the topsheet 24 and the backsheet 26. For unitary absorbent articles, the chassis 22 comprises the main structure of the diaper with other features added to form the composite diaper structure. While the topsheet 24, the backsheet 26, and the absorbent core 28 may be assembled in a variety of well-known configurations, preferred diaper configurations are described generally in U.S. Pat. No. 3,860,003 entitled "Contractible Side Portions for Disposable Diaper" issued to Kenneth B. Buell on January 14, 1975; U.S. Pat. No. 5,151,092 issued to Buell on September 9, 1992; and U.S. Pat No. 5,221,274 issued to Buell on June 22, 1993; and U.S. Pat, No. 5,554,145 entitled "Absorbent Article With Multiple Zone Structural Elastic-Like Film Web Extensible Waist Feature" issued to Roe et al. on September 10, 1996; U.S. Pat. No. 5,569,234 entitled "Disposable Pull-On Pant" issued to Buell et al. on October 29, 1996; U.S. Pat, No. 5,580,411 entitled "Zero Scrap Method For Manufacturing Side Panels For Absorbent Articles" issued to Nease, et al. on December 3, 1996; and U.S. Patent No. 6,004,306 entitled "Absorbent Article With Multi-Directional Extensible Side Panels" issued to Robles et al. on December 21, 1999.

The topsheet 24 may be fully or partially elasticized or may be foreshortened so as to provide a void space between the topsheet 24 and the core 28. Exemplary structures including elasticized or foreshortened topsheets are described in more detail in U.S. Pat No. 4,892,536 issued to DesMarais et al. on January 9, 1990 entitled "Absorbent Article Having Elastic Strands"; U.S. Pat. No. 4,990,147 issued to Freeland on February 5, 1991 entitled "Absorbent Article With Elastic Liner For Waste Material Isolation"; U.S. Pat. No. 5,037,416 issued to Allen et al. on August 6, 1991 entitled "Disposable Absorbent Article Having Elastically Extensible Topsheets"; and U.S. Pat. No. 5,269,775 issued to Freeland et al. on December 14, 1993 entitled "Trisection Topsheets For Disposable Absorbent Articles and Disposable Absorbent Articles Having Such Trisection Topsheets".

The absorbent core 28 may comprise any absorbent material that is generally compressible, conformable, non-irritating to the wearer's skin, and capable of absorbing and retaining liquids such as urine and other certain body exudates. The absorbent core 28 may comprise a wide variety of liquid-absorbent materials commonly used in disposable diapers and other absorbent articles such as comminuted wood pulp, which is generally referred to as air felt. Examples of other suitable absorbent materials include creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

Exemplary absorbent structures for use as the absorbent assemblies are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735, entitled "High Donsity Absorbent Members Having Lower Density and Lower Basis Weight Acquisition Zones", issued to Alemany et al. on May 30, 1989, U.S. Patent 4,888,231 entitled "Absorbent Core Having A Dusting Layer" issued to Angstadt on December 19, 1989; U.S. Patent No. 5,137,537 entitled "Absorbent Structure Containing Individualized, Polycarboxylic Acid Crosslinked Wood Pulp Cellulose Fibers" which issued to Herron et al. on August 11,1992; U.S. Patent 5,147,345 entitled "High Efficiency Absorbent Articles For Incontinence Management" issued to Young et al. on September 15, 1992; U.S. Patent No. 5,342,338 entitled "Disposable Absorbent Article For Low-Viscosity Fecal Material" issued to Roe on August 30, 1994; U.S. Patent No. 5,260,345 entitled "Absorbent Foam Materials For Aqueous Body Fluids and Absorbent Articles Containing Such Materials" issued to DesMarais et al. on November 9, 1993; U.S. Patent No. 5,387,207 entitled "Thin-Until-Wet Absorbent Foam Materials For Aqueous Body Fluids And Process For Making Same" issued to Dyer et al. on February 7, 1995; U.S. Pat. No. 5,397,316 entitled "Slitted Absorbent Members For Aqueous Body Fluids Formed Of Expandable Absorbent Materials" issued to LaVon ct al. on March 14, 1995; and U.S. Patent No. 5,625,222 entitled "Absorbent Foam Materials For Aqueous Fluids Made From high Internal Phase Emulsions Having Very High Water-To-Oil Ratios" issued to DesMarais et al. on July 22, 1997.

The backsheet 26 is generally that portion of the diaper 20 positioned adjacent the garment-facing surface of the absorbent core 28. Backsheet 26 prevents the exudates absorbed and contained therein from soiling articles that may contact the diaper 20, such as bed sheets and undergarments. In preferred embodiments, the backsheet 26 is substantially impermeable to liquids (e.g., urine) and comprises a laminate of a nonwoven and a thin plastic film such as a thermoplastic film having a thickness of about 0.012 mm (0.5 mil) to about 0.051 mm (2.0 mils). Suitable backsheet films include those manufactured by Tredagar Industries Inc. of Terre Haute, IN and sold under the trade names X15306, X10962, and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation BXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746, published on June 22, 1995 in the name of E.I. DuPont and copending U.S. Patent Application Serial No. 08/744,487, filed on November 6, 1996 in the name of Curro. Other breathable backsheets including nonwoven webs and apertured formed films are described in U.S. Patent No. 5,571,096 issued to Dobrin et al. on November 5, 1996. An exemplary, suitable backsheet is disclosed in U.S. Patent No. 6,107,537 entitled "Disposable absorbent articles providing a skin condition benefit" issued to Elder et al on August 22, 2000. Other suitable materials and/or manufacturing techniques may be used to provide a suitable backsheet 26 including, but not limited to, surface treatments, particular film selections and processing, particular filament selections and processing, etc.

Backsheet 26 may also consist of more than one layer, as exampled in FIG. 1, wherein a backsheet outer layer 26 (often referred to as the backsheet) may be made of a soft, nonwoven material and a backsheet inner layer 27 may be made of a substantially impermeable film. Adhesive 29, or any other suitable material or method, may be used to join layers 26 and 27 together. While a variety of backsheet configurations are contemplated herein, it would be obvious to those skilled in the art that various other changes and modifications can be made.

The diaper 20 may also include a fastening system 55. The fastening system 55 preferably maintains the first waist region 36 and the second waist region 38 in a configuration so as to provide lateral tensions about the circumference of the diaper 20 to hold the diaper 20 on the wearer. The fastening system 55 preferably comprises a fastener such as tape tabs, hook and loop fastening components, interlocking fasteners such as tabs & slots, buckles, buttons, snaps, and/or hermaphroditic fastening components, although any other known fastening means are generally acceptable. Some exemplary surface fastening systems are disclosed in U.S. Patent 3,848,594 entitled "Tape Fastening System for Disposable Diaper" issued to Buell on November 19, 1974; U.S. Patent B1 4,662,875 entitled "Absorbent Article" issued to Hirotsu et al. on May 5, 1987; U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestogard on January 16, 1990; U.S. Patent 4,946,527 entitled "Pressure-Sensitive Adhesive Fastener And Method of Making Same" issued to Battrell on August 7, 1990; the herein before referenced U.S. Pat. No. 5,151,092 issued to Buell on September 9, 1992; and U.S. Pat. No. 5,221,274 issued to Buell on June 22, 1993. An exemplary interlocking fastening system is disclosed in co-pending U.S. Patent No. 6,432,098 entitled "Absorbent Article Fastening Device" in the names of Kline et al, issued on August 13, 2002. The fastening system 55 may also provide a means for holding the article in a disposal configuration as disclosed in U.S. Pat. No. 4,963,140 issued to Robertson ct al. on October 16, 1990. The fastening system may also include primary and secondary fastening systems, as disclosed in U.S. Pat. No. 4,699,622 entitled "Disposable Diaper Having An Improved Side Closure" issued to Toussant et al. on October 13, 1987. to reduce shifting of overlapped portions or to improve fit as disclosed in U.S. Pat. No. 5,242,436 entitled "Absorbent Article With Fastening System Providing Dynamic Elasticized Waistband Fit" issued to Weil et al. on September 7, 1993; U.S. Pat. No. 5,499,978 entitled "Absorbent Article With Dynamic Elastic Waist Feature Having A Predisposed Resilient Flexural Hinge" issued to Buell et al. on March 19, 1996; U.S. Pat. No. 5,507,736 entitled "Absorbent Article With Dynamic Elastic Waist Feature Comprising An Expansive Tummy Panel" issued to Clear et al. on April 16, 1996; U.S. Pat. No. 5,591,152 entitled "Absorbent Article With Dynamic Elastic Waist Feature Having A Predisposed Resilient Flexural Hinge" issued to Buell et al. on January 7, 1997.

In alternative embodiments, the article may be preformed by the manufacturer to create a pant. The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). While the term "pant" is used herein, pants are also commonly referred to as "closed diapers", "prefastened diapers", "putl-on diapers", "training pants" and "diaper-pants". Suitable pants are disclosed in U.S. Patent No. 5,246,433, issued to Hassle, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Compel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The diaper 20 may also include such other features as are known in the art including cuffs, front and rear ear panels, waist cap features, elastics and the like to provide better fit, containment and aesthetic characteristics. Such additional features are well known in the art and are described in U.S. Pat. No. 3,860,003; and U.S. Patent No. 5,151,092.

For example, diaper 20 may include barrier cuffs or "stand-up" elasticized flaps, as exampled in U.S. Pat. Nos. 4,808,178 and 4,909,803 issued to Aziz et al. on Feb. 28, 1989 and Mar. 20, 1990, respectively.

Additionally, diaper 20 may include gasketing cuffs or "leg cuff" elasticized side flaps, as exampled in U.S. Pat, No. 3,860,003.

A first and second cuff may both be provided by way of a dual cuff, as exampled in U.S. Pat. Nos. 4,695,278 and 4,795,454 issued to Lawson on Sep. 22, 1987 and to Dragoo on Jan. 3, 1989, respectively.

For example, diaper 20 may include a first cuff 40 which provides improved containment of liquids and other body exudates. First cuff 40 may also be referred to as a barrier leg cuff, inner leg cuffs or "stand-up" elasticized flap. U.S. Pat. Nos. 4,808,178 and 4,909,803 issued to Aziz et al. on Feb. 28, 1989 and Mar. 20, 1990, respectively, describe disposable diapers having "stand-up" elasticized flaps that improve the containment of the leg regions.

Additionally, diaper 20 may include a second cuff 50 which also provides improved containment of liquids and other body exudates. Second cuff 50 may also be referred to as an outer leg cuff, leg band, side flap, leg cuff or elastic cuff, U.S, Pat. No. 3,860,003 describes a disposable diaper which provides a contractible leg opening having a side flap and one or more elastic members to provide an elasticized leg cuff.

First cuff 40 and second cuff 50 may both be provided by way of a dual cuff 30, as exampled in U.S. Pat. Nos. 4,695,278 and 4,795,454 issued to Lawson on Sep. 22, 1987 and to Dragoo on Jan. 3,1989, respectively.

FIG. 2 is a fragmentary sectional view taken along section line 2-2 of FIG. 1 and depicts the diaper construction in the crotch region 37 of the diaper 20 as it is shaped before being applied to the wearer (i.e., the diaper is subjected to elastic contraction). Dual cuff 30 has a proximate end 35, distal end 39 and regions therebetween identified as first cuff 40 and second cuff 50. Dual cuff 30 may be constructed from a continuous cuff material 33 that substantially envelopes the elastics of first cuff 40 and second cuff 50. More specifically, first cuff 40 has at least one elastic (although two elastics 42, 44 are shown) and second cuff 50 also has at least one elastic (although two elastics 52, 54 are shown) that are enveloped within cuff material 33. In this way, only a continuous cuff material 33 is used and manipulated during the construction of dual cuff 30, thus making easier the manufacturing of said dual cuff. Moreover, cuff material 33 need only be enclosed/bonded at a single location, as exampled by cuff bond 70, in order to substantially envelope elastics 42, 44, 52, 54, thus providing improved barrier properties by minimizing the number of potential leakable locations (e.g., bonding locations). Herein, "continuous cuff material" means a cuff material that is continuous along a path beginning from the cuff end bond, along said cuff material, and ending at the same cuff end bond such that the dual cuff 30 is substantially constructed of two layers of the materials, whether it be the same or different materials. For example, the continuous cuff material 33 may be constructed of a lessor-water-permeable material (e.g., spunbound material which is inexpensive) with a more-water-permeable material (e.g., meltblown material which is more expensive) placed inside said lesser-water-permeable material. In another example, cuff material 33 may be constructed of a spunbound-meltblown laminate. In yet another example, cuff material 33 may be constructed of a series of various materials so long as they are continuous. In yet another example, cuff material 33 may be treated to increase its hydrophobicity. Such hydrophobic treatments include, but are not limited to, the application of hydrophobic surface coating (as exampled in co-pending U.S. Patent Application Serial No. 60/543,785, entitled "Hydrophobio Surface Coated Absorbent Articles And Associated Methods", filed on February 11, 2004) and flouro-treatment (as exampled in copending U.S. Patent Application Serial No. 10/703,239, entitled "Disposable Absorbent Articles With Masking Topsheet", filed on November 7, 2003). Dual cuff 30 is connected to diaper 20 by way of a single bond (e.g., adhesive, ultrasonic; e.g., cuff bond 70 to topsheet 24).

Cuff bond 70 may extend substantially the entire longitudinal length of diaper 20. Cuff bond 70 may be adhesive, ultrasonic bonding, compression bonding, thermal bonding, combinations thereof, and any other suitable bonding means known in the art which is appropriate for the specific materials employed. Cuff bond 70 may also join dual cuff 30 to topsheet 24. Lastly, an adhesive 76 having liquid impermeability properties may be applied between the topsheet 24 and backsheet 26 (or more specifically shown herein, backsheet inner layer 27) to provide improved barrier properties. Adhesive 76 may be located juxtaposed to intermediate bond 70; however, so long as adhesive 76 helps to provide a containment of exudates, then its actual location is may be variable. The cuff material is only bonded to itself by the cuff bond (i.e., no other bond similar to cuff bond 70 is used). Lastly, it may be desirable for both first cuff 40 and second cuff 50 to be barrier cuffs.

Elastics 42, 44, 52, 54 may be operatively associated with their respective cuff by securing it within said cuff with an elastic attachment element 46. The elastic attachment element 46 should be flexible and of sufficient adhesiveness to hold elastics 42, 44, 52, 54 in their stretched condition. Elastics 42, 44, 52, 54, having a first and second end, may be secured to their respective cuff only near their ends or along their entire length. Elastic attachment clement 46 may be glue beads made of hot melt adhesive such as marketed by Findley Adhesives Incorporated, Elmgrove, Wis., as Findley Adhesives 581. Alternatively, elastic attachment element 46 may take the form of an ultrasonic bond or heat/pressure seal. A more detailed description of the manner in which the elastic attachment element 46 may be positioned and secured to their respective cuff can be found in U.S. Pat. No. 4,081, 301, issued to Buell on Mar. 28, 1978, and in U.S. Pat. No. 4,253,461, issued to Strickland and Visscher on Mar. 3, 1981. While elastics 42, 44 in first cuff 40 and elastics 52, 54 in second cuff 50 were shown, it would be obvious to those skilled in the art that one or more elastics may be used in each cuff.

Elastics 42, 44, 52, 54 which have been found suitable are elastic strands having a cross section of 0.18 mm by 1.5 mm and made from natural rubber as available from Easthampton Rubber Company of Stewart, Va., under the trademark L-1900 Rubber Compound. Other suitable elastics can be made from natural rubber, such as elastic tape sold under the trademark Fulflex 9411 by Fulflex Company of Middletown, R. I. Elastics 42, 44, 52, 54 may also comprise any heat shrinkable elastic material as is well known in the art. Other suitable elastic materials may comprise a wide variety of materials as are well known in the art include elastomeric films, polyurethane films, elastomeric foams, formed elastic scrim and synthetic elastomers (e.g., Lycra™). In addition, elastics 42, 44, 52, 54 may take a multitude of configurations. For example, the width may be varied; a single strand or several parallel or non-parallel strands of elastic material may be used; or a variety of shaped may be used including rectilinear and curvilinear.

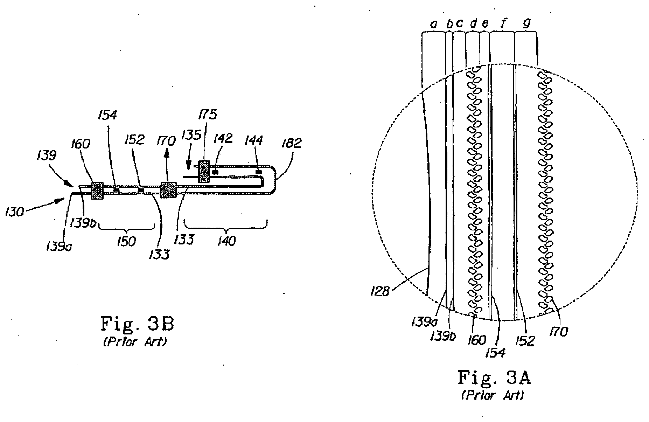

Referring now to FIG. 3a, a top view taken within encircled portion 3 of FIG. 1 is shown illustrating a prior art example of a dual cuff 130 within the crotch region 37. FIG 3b depicts a corresponding cross-sectional view of FIG. 3a. Prior art dual cuff 130 is constructed from at least two cuff materials 133 joined together by bonds 160, 170, 175. Prior art dual cuff 130 has a proximate end 135, distal end 139 and regions therebetween identified as first cuff 140 and second cuff 150. More specifically, first cuff 140 has two elastics 142, 144 and second cuff 150 also has two elastics 152, 154 that are enveloped within cuff materials 133.

When measuring the overall width of an absorbent article 20, the widths of dual cuff elements and the distances between each element are considered. For example, referring to FIG. 3a, distance "a" is the length measured from longitudinal edge 128 to cuff distal edge 139a. As background, near the end of the manufacturing process, a side notch cut is made into the backsheet to create an overall hour glass shape to provide leg openings within the crotch region. When making said side notch cut, it is important that the rotary knife, for example, does not inadvertently extend into cuff distal and 139a; otherwise, a perforated piece results that may later be removed by the wearer (e.g., baby) thus causing a safety concern of removing and swallowing said piece. Therefore, in effect, distance "a" is a safety standard against this potential problem. Based on the tracking inaccuracies of a traveling web of material being fed into a rotary knife in the manufacturing of an absorbent article, distance "a" is commonly set at approximately 7mm.

Distance "b" is the length measured from cuff distal edge 139a to cuff distal edge 139b. More specifically, since prior art dual cuff 130 is made by two cuff materials 133 that are joined together by a cuff end bond 160, there exists misalignment issues in placing edges 139a and 139b next to one another. This distance "b" is an inherent property of this prior art dual cuff design and typically measures at approximately 2mm.

Distance "c" is the length measured from cuff distal edge 139b to cuff end bond 160. More specifically, cuff end bond 160 is intended to join cuff distal edges 139a, 139b. To ensure that cuff distal edges 139a, 139b are in fact joined in a bonding roll, and based on the tracking inaccuracies of a traveling web of material being fed into a bonding roll in the manufacturing of an absorbent article, distance "c" is commonly set at approximately 3mm.

Distance "d" is the actual width of cuff end bond 160. While the widths of elastics 142, 144, 152, 154 are comparatively insubstantial, the width of cuff end bond is substantial and typically measures at 2mm. This distance "d" is an inherent property of this prior art dual cuff design.

Distance "e" is the length measured from cuff end bond 160 to second cuff elastic 152. To ensure that second cuff elastic 152 does not inadvertently get bonded by cuff end bond 160, and based on the tracking inaccuracies of a traveling web of material being fed into a bonding roll in the manufacturing of an absorbent article, distance "e" is commonly set at approximately 3mm.

Distance "f" is the length measured from second cuff elastic 152 to second cuff elastic 154. Both elastics 152, 154 are typically applied with adhesive to the traveling web of material. If elastics 152, 154 having adhesive (not shown) come in contact with one another, then they may inadvertently join, thus requiring the stopping and repairing of the manufacturing process. Given the dramatic impact of shutting down the manufacturing process, distance "f" is safely set at 5mm. Said distance may also be important for the proper functioning of said second cuff generally.

Distance "g" is the length measured from second cuff elastic 154 to cuff intermediate bond 170. More specifically, cuff intermediate bond 170 is intended to join dual cuff 130 to the topsheet (see FIG. 2). To ensure that second cuff elastic 154 does not inadvertently get bonded by cuff intermediate bond 170, and based on the tracking inaccuracies of a traveling web of material being fed into a bonding roll in the manufacturing of an absorbent article, distance "g" is commonly set at approximately 3mm.

Consequently, the summation of said widths for dual cuff 130 is equal to 25mm. Next, recognizing that there are two dual cuffs 130 per absorbent article (i.e., one on each side), the two dual cuffs 130 account for 50mm of the overall width of said article. Lastly, assume a common distance between said dual cuffs of 115mm. Thus, the overall width of the prior art absorbent article within the crotch region is equal to 165mm.

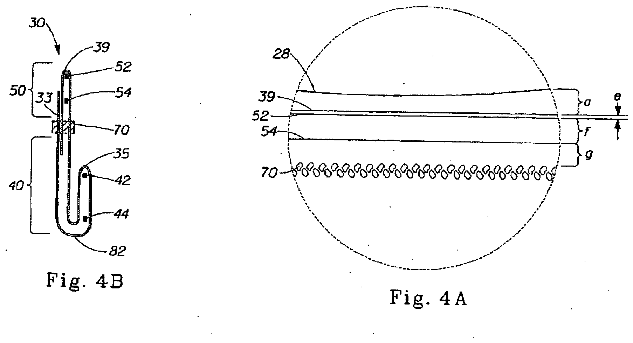

Referring now to FIG. 4a, a top view taken within encircled portion 3 of FIG. 1 is shown illustrating a dual cuff 30 within the crotch region in accordance with the present invention, FIG 4b depicts a corresponding cross-sectional view of FIG. 4a. Dual cuff 30 is constructed from a continuous cuff material 33 which is enclosed by cuff bond 70. Dual cuff 30 has a proximate end 35, distal end 39 and regions therebetween identified as first cuff 40 and second cuff 50. More specifically, first cuff 40 has at least one elastic (although two elastics 42, 44 are shown) and second cuff 50 also has at least one elastic (although two elastics 52, 54 are shown) that are enveloped within cuff material 33.

In comparing the overall width of the absorbent article in FIG. 4a (present invention) to FIG. 3a (prior art), a similar analysis is conducted. For example, referring to FIG. 4a, distance "a" is substantially the same length as that provided in FIG. 3a; however, the length is measured from longitudinal edge 28 to cuff distal edge 39 (as compared to 139a). Because the same safety and manufacturing considerations exist, distance "a" is commonly set at approximately 7mm.

Distance "b" (measured in FIG. 3a as the length measured from cuff distal edge 139a to cuff distal edge 139b) does not exist in FIG. 4a because a continuous cuff material 33 is folded to create cuff distal edge 39, rather than two cuff materials juxtaposed and bonded. Therefore, distance "b" is negligible.

Distance "c" (measured in FIG. 3a as the length measured from cuff distal edge 139b to cuff end bond 160) does not exist in FIG. 4a because the folded, continuous cuff material 33 does not need to be bonded in such close proximity to cuff distal edge 139. Therefore, distance "c" is negligible.

Distance "d" (measured in FIG. 3a as the actual width of cuff end bond 160) does not exist in FIG. 4a because no such bond in such close proximity to cuff distal edge 39 is needed. Therefore, distance "d" is negligible.

Distance "e" is substantially the same measurement as that provided in FIG. 3a; however, the length is measured from cuff distal edge 39 (as compared to cuff end bond 160) to second cuff elastic 52. Since cuff distal edge 39 is folded about second cuff elastic 52, distance "e" is negligible.

Distance "f" is substantially the same length (i.e., measured from second cuff elastic 52 to second cuff elastic 54) as that provided in FIG. 3a. Therefore, distance "f' is similarly set at 5mm.

Distance "g" is substantially the same length (i.e., measured from second cuff elastic 54 to cuff bond 70) as that provided in FIG. 3a. Therefore, distance "f" is similarly set at 3mm.

Consequently, the summation of said widths for dual cuff 30 is equal to 15mm. Next, recognizing that there are two dual cuffs 30 per absorbent article (i.e., one on each side), the two dual cuffs 30 account for 30mm of the overall width of said article. Lastly, assume a common distance between said dual cuffs of 115mm. Thus, the overall width of the prior art absorbent article within the crotch region is equal to 145mm.

In comparing the overall width of the FIG. 3a (prior art; measuring at 165mm) and FIG. 4a (present invention; measuring at 145mm), the difference is 20mm. This reduction in overall width within the crotch region has been discovered to provide significant comfort and wear properties. For example, toddlers wearing diapers in accordance with the present invention walk more naturally because the crotch region is narrower and thus said diaper does not significantly interfere with the inner legs of the wearer. Furthermore, caregivers, and sometimes the toddlers themselves, prefer the diaper to have a narrower crotch because it aesthetically looks more like underwear. This latter product benefit is especially important as toddlers learn about the differences between diapers and underwear (i.e., potty-training).

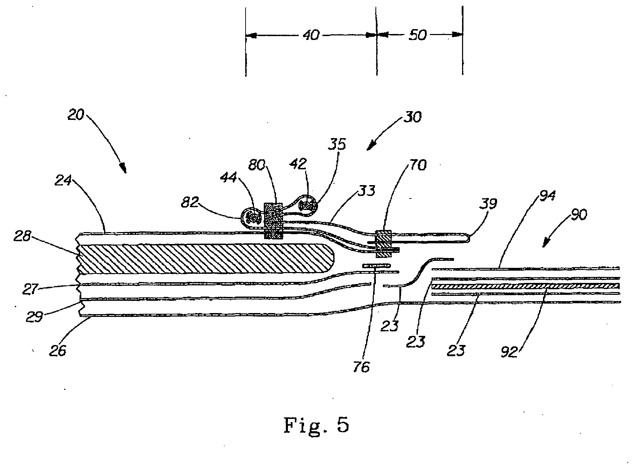

FIG. 5 is a fragmentary sectional view taken along section line 5-5 of FIG. 1 and depicts the diaper construction in the first waist region 36 of the diaper 20 as it is shaped before being applied to the wearer (i.e., the diaper is subjected to elastic contraction). First cuff 40 is laid down (e.g., folded to create cuff fold 82) and joined to topsheet 24 so as to more readily confirm to the wearer's waist region. First cuff 40 may be joined to topsheet 24 by a cuff tuckdown bond 80 which may be adhesive, ultrasonic bonding, compression bonding, thermal bonding, combinations thereof, and any other suitable bonding means known in the art which is appropriate for the specific materials employed.

Additionally, side panels 90 may be provided in first and second waist regions 36, 38 and adjacent to longitudinal edges 14. Side panels 90 may comprise a side panel elastic 92 and a side panel cover 94, wherein, the side panel elastic 92 is positioned between said side panel cover 94 and backsheet 26. Adhesive 23, or any other suitable material or method, may be used to join these components together and to cuff distal end 39.

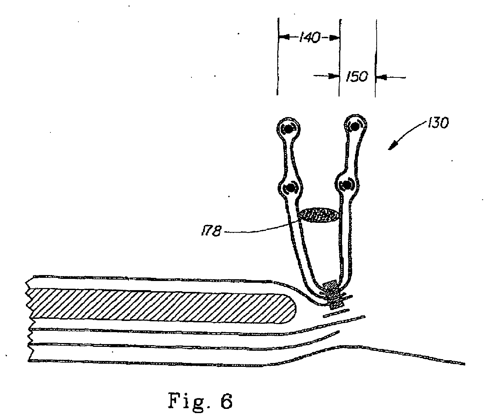

FIG. 6 is a fragmentary sectional view taken along section line 2-2 of FIG. 1 and depicts another non-limitmg, exemplary embodiment of a diaper construction in the crotch region 37 of a diaper as it is shaped before being applied to the wearer (i.e., the diaper is subjected to elastic contraction). Dual cuff 130 has a first cuff 140 and second cuff 150 that are joined together by adhesive 178 or any other suitable material/bonding technique. In this way, dual cuff 130 stands taller and thus may provide better exudate containment. While only a single location of adhesive 178 is shown, one skilled in the art would appreciate that multiple locations and configurations of adhesive application may be used.

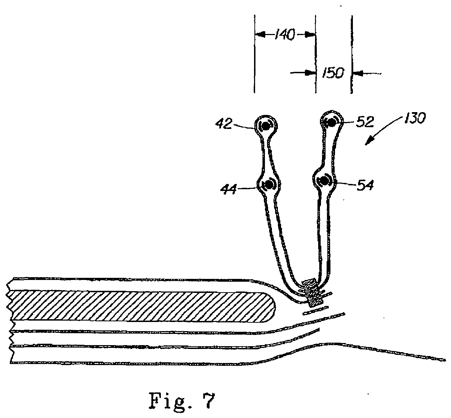

FIG. 7 is a fragmentary sectional view taken along section line 2-2 of FIG. 1 and depicts another non-limiting, exemplary embodiment of a diaper construction in the crotch region 37 of a diaper as it is shaped before being applied to the wearer (i.e., the diaper is subjected to elastic contractivn). Second cuff 150 may be constructed to stand substantially upright without the aid of additional elements (e.g., adhesive 178). For example, elastic 52 and/or elastic 54 may be constructed of a material having a larger diameter than that of elastics 42, 44. In another example, elastics 52 and/or elastic 54 may be applied having greater tension. In yet another example, elastic 52 may be continuously bonded and elastic 54 may be bonded near its ends to create a drawstring. One skilled in the art would also appreciate that such techniques may be applied to first cuff 140 and its respective elastics 42, 44.

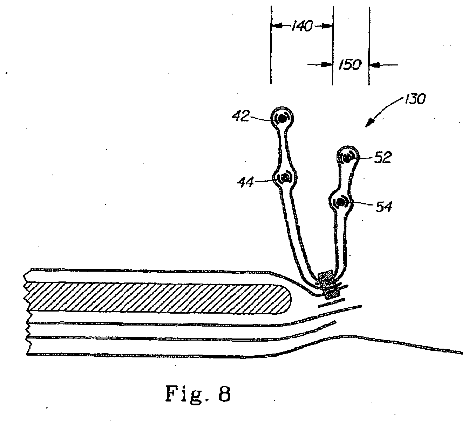

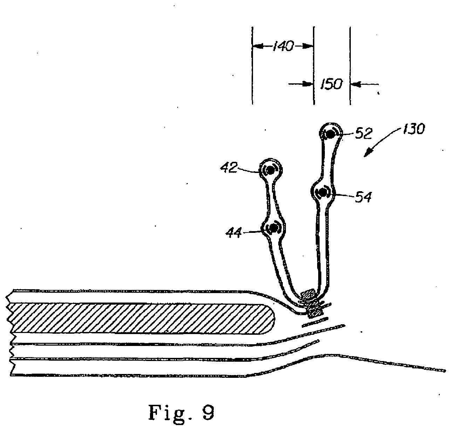

FIG. 8 and FIG. 9 are fragmentary sectional views taken along section line 2-2 of FIG. 1 and depict exemplary embodiments of a diaper construction in the crotch region 37 of a diaper as it is shaped before being applied to the wearer (i.e., the diaper is subjected to elastic contraction). More specifically, FIG. 8 shows an exemplary embodiment wherein first cuff 140 stands taller than second cuff 150. Conversely, FIG. 9 shows an exemplary embodiment wherein second cuff 150 stands taller than first cuff 140.

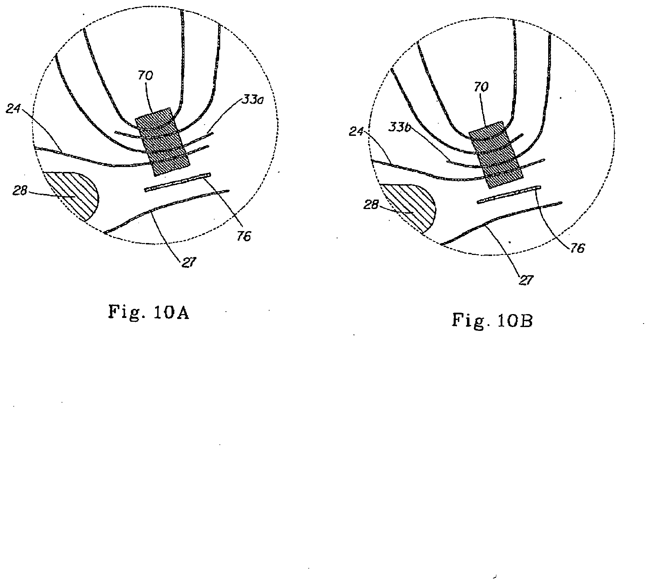

FIG. 10a is a close-up view taken within encircled portion 10 of FIG. 2 illustrating a fold-over technique of cuff material, wherein a lower cuff material end 33a is positioned longitudinally outboard of cuff bond. Alternatively, FIG. 10b is a close-up view taken within encircled portion 10 of FIG. 2 illustrating a fold-over technique of cuff material, wherein a lower cuff material end 33a is positioned longitudinally inboard of cuff bond. While both fold-over techniques are suitable for the present invention, the technique of FIG. 10a is preferable in that the cuff material may be cut on-line: whereas, the technique of FIG. 10b requires the cuff material to be pre-cut and then subsequently handled.

Embodiments of the present invention may also include pockets for receiving and containing waste, spacers which provide voids for waste, barriers for limiting the movement of waste in the article, compartments or voids which accept and contain waste materials deposited in the diaper 20, and the like, or any combinations thereof. Examples of pockets and spacers for use in absorbent products are described in U.S. Patent 5,514,121 issued to Roc et al. on May 7, 1996, entitled "Diaper Having Expulsive Spacer"; U.S. Patent 5,171,236 issued to Dreier et al. on December 15, 1992 entitled "Disposable Absorbent Article Having Core Spacers"; U.S. Patent 5,397,318 issued to Dreier on March 14, 1995 entitled "Absorbent Article Having A Pocket Cuff'; U.S. Patent 5,540,671 issued to Dreier on July 30,1996 entitled "Absorbent Article Having A Pocket Cuff With An Apex"; PCT Application WO 93/25172 published December 3, 1993 entitled "Spacers For Use In Hygienic Absorbent Articles And Disposable Absorbent Articles Having Such Spacer"; U.S. Patent 5,306,266 entitled "Flexible Spacers For Use In Disposable Absorbent Articles" issued to Freeland on April 26, 1994; and U.S. Patent 5,997,520 entitled "Disposable Absorbent Article With Selectively Expandable or Inflatable Component" issued to Ahr et al. on December 7, 1999. Examples of compartments or voids are disclosed in U.S. Patent 4,968,312 entitled "Disposable Fecal Compartmenting Diaper" issued to Khan on November 6, 1990; U.S. Patent 4,990,147 entitled "Absorbent Article With Elastic Liner For Waste Material Isolation" issued to Freeland on February 5, 1991; U.S. Patent 5,062,840, entitled "Disposable Diapers" issued to Holt et al on November 5, 1991; and U.S. Patent 5,269,755 entitled "Trisection Topsheets For Disposable Absorbent Articles And Disposable Absorbent Articles Having Such Trisection Topsheets" issued to Freeland et al on December 14, 1993. Examples of suitable transverse barriers are described in U.S. Pat. No. 5,554,142 entitled "Absorbent Article Having Multiple Effective Height Transverse Partition" issued September 10, 1996 in the name of Dreier et al.; PCT Patent WO 94/14395 entitled "Absorbent Article Having An Upstanding Transverse Partition" published July 7, 1994 in the name of Freeland, et al.; and U.S. Patent No. 5,653,703 Absorbent Article Having Angular Upstanding Transverse Partition issued Aug. 5, 1997 to Roe, et al. Examples of other structures especially suitable for management of low viscosity fences are disclosed in U.S. Patents 5,941,864 issued to Roe et al. on August 24, 1999; U.S. Patent No. 5,977,430 issued to Roe et al. on Nov. 2, 1999 and 6,013,063 issued to Roe et al. on January 11, 2000.

In addition, the present invention may be suitable for other diaper embodiments including those disclosed in U.S. Patent No. 6,010,491 titled "'Viscous Fluid Bodily Waste Management Article" issued January 4, 2000; U.S. Patent No. 5,873,870 titled "Fit And Sustained Fit Of A Diaper Via Chassis And Core Modifications" issued February 23, 1999; U.S. Patent No. 5,897,545 titled "Elastomeric Side Panel for Use with Convertible Absorbent Articles" issued April 27, 1999; U.S. Patent No. 5,904,673 titled "Absorbent Article With Structural Elastic-Like Film Web Waist Belt" issued May 18, 1999; U.S. Patent No. 5,931,827 titled "Disposable Pull On Pant" issued August 3, 1999; U.S. Patent No. 5,977,430 titled "Absorbent Article With Macro-Particulate Storage Structure" issued November 2, 1999 and U.S. Patent No. 6,004,306 titled "Absorbent Article With Multi-Directional Extensible Side Panels" issued December 21, 1999.

## Claims

1. A unitary disposable absorbent article in the form of a disposable diaper, the article having a longitudinal axis (1000) and a lateral axis (1100), opposite longitudinal end portions of the article being configured as respective first and second waist regions (36, 38), and an intermediate portion of the article being configured as a crotch region (37) which extends longitudinally between the first and second waist regions (36, 38), the article comprising:
an absorbent core (28) having a garment surface and a body surface;
a liquid permeable topsheet (24) positioned adjacent said body surface of said absorbent core (28);
a liquid impermeable backsheet (26) positioned adjacent said garment surface of said absorbent core (28); and
an elastically contractible dual cuff (30) having a proximate end (35) and a distal end (39), said dual cuff (30) being joined to said article by a cuff bond (70), said dual cuff (30) having a first cuff (40) and a second cuff (50), said first cuff (40) being disposed between said proximate end (35) and said cuff bond (70), said second cuff (50) being disposed between said cuff bond (70) and said distal end (39), said dual cuff (30) being constructed of a continuous cuff material (33) and enclosed by said cuff bond (70),
wherein said distal (39) end is formed by said cuff material being folded, wherein said proximate (35) end is formed by said cuff material being folded;
wherein said first cuff (40) envelopes at least one first elastic, wherein said first elastic has a first and second end, wherein said first elastic is secured to said first cuff near said first and second ends;
wherein said second cuff (50) envelopes at least one second elastic, wherein said second elastic has a first and second end, wherein said second elastic is secured to said second cuff near said first and second ends;
**characterized in that** said first and second cuffs are barrier cuffs, and **in that** said dual cuff (30) is bonded to said article in said crotch region (37) by a single bond, wherein said single bond is said cuff bond (70),
and wherein said cuff material (33) is enclosed in said crotch region (37) by being bonded to itself by the cuff bond (70), said cuff material (33) in said crotch region (37) only being bonded to itself by the cuff bond (70).

2. The absorbent article of claim 1 wherein said first cuff (40) envelops at least one elastic.

3. The absorbent article of claim 2 wherein said elastic is operatively associated with said first cuff (40) by securing it with an elastic attachment element (46).

4. The absorbent article of claim 1 wherein said first cuff (50) envelops at least one elastic.

5. The absorbent article of claim 1 wherein said elastic is operatively associated with said second cuff (50) by securing it with an elastic attachment element (46).

6. The absorbent article of claim 1 wherein said disposable diaper is a prefastened diaper.

7. The absorbent article of claim 1 wherein said continuous cuff material (33) is constructed of a lesser-water-permeable material with a more-water-permeable material placed inside said lesser-water-permeable material.

## Patentansprüche

1. Einstückiger Einweg-Absorptionsartikel in der Form einer Einwegwindel, wobei der Artikel eine Längsachse (1000) und eine Querachse (1100) aufweist, wobei gegenüberliegende longitudinale Endabschnitte des Artikels als jeweiliger erster und zweiter Taillenbereich (36, 38) konfiguriert sind, und ein zwischenliegender Abschnitt des Artikels als ein Schrittbereich (37) konfiguriert ist, der sich längs zwischen dem ersten und dem zweiten Taillenbereich (36, 38) erstreckt, wobei der Artikel umfasst:
einen Absorptionskern (28) mit einer bekleidungsseitigen Oberfläche und einer körperseitigen Oberfläche;
eine flüssigkeitsdurchlässige Oberschicht (24), die angrenzend an die körperseitige Oberfläche des Absorptionskerns (28) positioniert ist;
eine flüssigkeitsundurchlässige Unterschicht (26), die angrenzend an die bekleidungsseitige Oberfläche des Absorptionskerns (28) positioniert ist; und
ein elastisch kontrahierbares Doppelbündchen (30) mit einem nahe gelegenen Ende (35) und einem entfernt gelegenen Ende (39), wobei das Doppelbündchen (30) an den Artikel durch eine Bündchenverbindung (70) angebracht wird, wobei das Doppelbündchen (30) ein erstes Bündchen (40) und ein zweites Bündchen (50) aufweist, wobei das erste Bündchen (40) zwischen dem nahe gelegenen Ende (35) und der Bündchenverbindung (70) angeordnet ist, wobei das zweite Bündchen (50) zwischen der Bündchenverbindung (70) und dem entfernt gelegenen Ende (39) angeordnet ist, wobei das Doppelbündchen (30) aus einem durchgängigen Bündchenmaterial (33) hergestellt ist und durch die Bündchenverbindung (70) eingeschlossen wird,
wobei das entfernt gelegene Ende (39) gebildet wird, indem das Bündchenmaterial gefaltet wird, wobei das nahe gelegene Ende (35) gebildet wird, indem das Bündchenmaterial gefaltet wird;
wobei das erste Bündchen (40) mindestens ein erstes Gummiband umschließt, wobei das erste Gummiband ein erstes und ein zweites Ende aufweist, wobei das erste Gummiband am ersten Bündchen nahe des ersten und zweiten Endes befestigt ist;
wobei das zweite Bündchen (50) mindestens ein zweites Gummiband umschließt, wobei das zweite Gummiband ein erstes und ein zweites Ende aufweist, wobei das zweite Gummiband am zweiten Bündchen nahe des ersten und zweiten Endes befestigt ist;
**dadurch gekennzeichnet, dass** das erste Bündchen und zweite Bündchen Sperrbündchen sind, und **dadurch, dass** das Doppelbündchen (30) in dem Schrittbereich (37) durch eine Einfachverbindung mit dem Artikel verbunden ist, wobei die Einfachverbindung die Bündchenverbindung (70) ist,
und wobei das Bündchenmaterial (33) in dem Schrittbereich (37) eingeschlossen ist, indem es durch die Bündchenverbindung (70) an sich selbst gebunden wird, wobei das Bündchenmaterial (33) in dem Schrittbereich (37) ausschließlich durch die Bündchenverbindung (70) an sich selbst gebunden ist.

2. Absorptionsartikel nach Anspruch 1, wobei das erste Bündchen (40) mindestens ein Gummiband umschließt.

3. Absorptionsartikel nach Anspruch 2, wobei das Gummiband funktionell mit dem ersten Bündchen (40) assoziiert wird, indem es mit einem elastischen Befestigungselement (46) befestigt wird.

4. Absorptionsartikel nach Anspruch 1, wobei das erste Bündchen (50) mindestens ein Gummiband umschließt.

5. Absorptionsartikel nach Anspruch 1, wobei das Gummiband funktionell mit dem zweiten Bündchen (50) verbunden wird, indem es mit einem elastischen Befestigungselement (46) befestigt wird.

6. Absorptionsartikel nach Anspruch 1, wobei die Einwegwindel eine vorverschlossene Windel ist.

7. Absorptionsartikel nach Anspruch 1, wobei das kontinuierliche Bündchenmaterial (33) aus einem weniger wasserdurchlässigen Material hergestellt ist, wobei ein stärker wasserdurchlässiges Material in dem weniger wasserdurchlässigen Material angeordnet ist.

## Revendications

1. Article absorbant jetable unitaire sous la forme d'une couche jetable, l'article ayant un axe longitudinal (1000) et un axe latéral (1100), les parties d'extrémité longitudinales opposées de l'article étant configurées sous la forme de première et seconde régions de ceinture (36, 38) respectives, et une partie intermédiaire de l'article étant configuré sous la forme d'une région d'entrejambe (37) qui s'étend longitudinalement entre les première et seconde régions de ceinture (36, 38), l'article comprenant :
une âme absorbante (28) ayant une surface de vêtement et une surface corporelle ;
une feuille de dessus perméable aux liquides (24) positionnée de façon adjacente à ladite surface corporelle de ladite âme absorbante (28) ;
une feuille de fond imperméable aux liquides (26) positionnée de façon adjacente à ladite surface de vêtement de ladite âme absorbante (28) ; et
un revers double élastiquement contractile (30) ayant une extrémité proximale (35) et une extrémité distale (39), ledit revers double (30) étant joint audit article par une liaison de revers (70), ledit revers double (30) ayant un premier revers (40) et un deuxième revers (50), ledit premier revers (40) étant disposé entre ladite extrémité proximale (35) et ladite liaison de revers (70), ledit deuxième revers (50) étant disposé entre ladite liaison de revers (70) et ladite extrémité distale (39), ledit revers double (30) étant construit dans un matériau de revers continu (33) et enclavé par ladite liaison de revers (70),
dans lequel ladite extrémité distale (39) est formée par ledit matériau de revers qui est plié, dans lequel ladite extrémité proximale (35) est formée par ledit matériau de revers qui est plié ;
dans lequel ledit premier revers (40) enveloppe au moins un premier élastique, dans lequel ledit premier élastique a une première et une deuxième extrémité, dans lequel ledit premier élastique est fixé audit premier revers près desdites première et deuxième extrémités ;
dans lequel ledit deuxième revers (50) enveloppe au moins un deuxième élastique, dans lequel ledit deuxième élastique a une première et une deuxième extrémité, dans lequel ledit deuxième élastique est fixé audit deuxième revers près desdites première et deuxième extrémités ;
**caractérisé en ce que** lesdits premier et deuxième revers sont des revers formant barrière, et **en ce que** ledit double revers (30) est collé sur ledit article dans ladite région d'entrejambe (37) par une liaison simple, ladite liaison simple étant ladite liaison de revers (70),
et dans lequel ledit matériau de revers (33) est enfermé dans ladite région d'entrejambe (37) en étant lié à lui-même par la liaison de revers (70), ledit matériau de revers (33) dans ladite région d'entrejambe (37) n'étant lié qu'à lui-même par la liaison de revers (70).

2. Article absorbant selon la revendication 1, dans lequel ledit premier revers (40) enveloppe au moins un élastique.

3. Article absorbant selon la revendication 2, dans lequel ledit élastique est associé opérationnellement audit premier revers (40) en le fixant avec un élément d'attachement élastique (46).

4. Article absorbant selon la revendication 1, dans lequel ledit premier revers (50) enveloppe au moins un élastique.

5. Article absorbant selon la revendication 1, dans lequel ledit élastique est associé opérationnellement audit deuxième revers (50) en le fixant avec un élément d'attachement élastique (46).

6. Article absorbant selon la revendication 1, dans lequel ladite couche jetable est une couche préattachée.

7. Article absorbant selon la revendication 1, dans lequel ledit matériau de revers continu (33) est construit en un matériau moins perméable à l'eau avec un matériau plus perméable à l'eau placé à l'intérieur dudit matériau moins perméable à l'eau.
